# EUROPEAN PATENT SPECIFICATION

(11) **EP 1 283 868 B1**
(45) Date of publication and mention of the grant of the patent: **29.07.2009**
(21) Application number: 01934259.1
(22) Date of filing: 07.05.2001
(51) Int. Cl.: C08B 37/00, C07H 1/00

(54) **ISOLATED GUM OPERON FROM XYLLELA FASTIDIOSA, ISOLATED NUCLEIC ACID MOLECULES THEREFROM, AND USES THEREOF**
ISOLIERTES GUM-OPERON AUS XYLLELA FASTIDIOSA, ISOLIERTE NUKLEINSÄUREMOLEKÜLE UND DEREN VERWENDUNGEN
OPERON DE GOMME ISOLE DE XYLLELA FASTIDIOSA, SES MOLECULES D'ACIDE NUCLEIQUE ISOLEES ET SES UTILISATIONS

(30) Priority: 09.05.2000 US 567465
(43) Date of publication of application: 19.02.2003
(73) Proprietor: Fundacao de Amparo a Pesquisa do Estado de Sao Paulo - FAPESP, CEP-05468-901 Sao Paulo, SP (BR)
(72) Inventor: Arruda, Paulo Universidade Estadual de Campinas, CEP-1308-970 Campinas, SP (BR); Da Silva, Felipe R. Uni. Estadual de Campinas, CEP-1308-970 Campinas, SP (BR); Kemper, Edson L. Universidade Estadual de Campinas, CEP-1308-970 Campinas, SP (BR); Vettore, Andre Universidade Estadual de Campinas, CEP-1308-970 Campinas, SP (BR); Leite, Adilson Universidade Estadual de Campinas, CEP-1308-970 Campinas, SP (BR); Da Silva, Marcio J. Uni. Estadual de Campinas, CEP-1308-970 Campinas, SP (BR)
(74) Representative: Weickmann, Franz Albert
(86) International application number: PCT/IB2001/001004
(87) International publication number: WO 2001/085905

(56) References cited:
- WO-A-87/05939
- WO-A-92/19753
- WO-A-98/56942
- LAMBAIS M R ET AL: "A GENOMIC APPROACH TO THE UNDERSTANDING OF XYLELLA FASTIDIOSA PATHOGENICITY" CURRENT OPINION IN MICROBIOLOGY, CURRENT BIOLOGY LTD, GB, vol. 3, 2000, pages 459-462, XP002909502 ISSN: 1369-5274
- DOW J M ET AL: "Xylella genomics and bacterial pathogenicity to plants" YEAST, vol. 17, no. 4, December 2000 (2000-12), pages 263-271, XP009035718 ISSN: 0749-503X
- MACHADO M A ET AL: "Genome and pathogenicity of Xylella fastidiosa" MOLECULAR BIOLOGY TODAY 2001 UNITED KINGDOM, vol. 2, no. 3, 2001, pages 33-43, XP001182847 ISSN: 1468-5698
- SIMPSON A J ET AL: "The genome sequence of the plant pathogen Xylella fastidiosa. The Xylella fastidiosa Consortium of the Organization for Nucleotide Sequencing and Analysis" NATURE, MACMILLAN JOURNALS LTD. LONDON, GB, vol. 406, no. 6792, 13 July 2000 (2000-07-13), pages 151-157, XP002224297 ISSN: 0028-0836
- KATZEN FEDERICO ET AL: "Xanthomonas campestris pv. campestris gum mutants: Effects on xanthan biosynthesis and plant virulence" JOURNAL OF BACTERIOLOGY, vol. 180, no. 7, April 1998 (1998-04), pages 1607-1617, XP002294015 ISSN: 0021-9193
- TAIT M I ET AL: "SYNTHESIS AND PROPERTIES OF A MUTANT TYPE OF XANTHAN" JOURNAL OF APPLIED BACTERIOLOGY, vol. 66, no. 5, 1989, pages 457-460, XP009035720 ISSN: 0021-8847
- LEVY SAMUEL ET AL: "Dynamic simulations of the molecular conformations of wild type and mutant xanthan polymers suggest that conformational differences may contribute to observed differences in viscosity" BIOPOLYMERS, vol. 38, no. 2, 1996, pages 251-272, XP001183435 ISSN: 0006-3525
- HASSLER R A ET AL: "GENETIC ENGINEERING OF POLYSACCHARIDE STRUCTURE PRODUCTION OF VARIANTS OF XANTHAN GUM IN XANTHOMONAS-CAMPESTRIS" BIOTECHNOLOGY PROGRESS, vol. 6, no. 3, 1990, pages 182-187, XP001182843 ISSN: 8756-7938
- PAULO ARRUDA: "Functional analysis of the xylella fastidiosa Gum Operon and its relation to the Citrus Variegated Clorosis (CVC)" INTERNET PUBLICATION, [Online] 4 November 1999 (1999-11-04), XP002294016 Retrieved from the Internet: <URL:http://web.archive.org/web/1999110410 1513/http://watson.fapesp.br/funcional/1ed ital/parruda.html> [retrieved on 2004-08-26]
- DATABASE NUCLEOTIDE 31 January 1996 (1996-01-31) BECKER, A. ET AL.: "Xanthomonas campestris GumA, GumB, GumC, GumD, GumE, GumF, GumG, GumH, GumI, GumJ, GumK, GumL and GumM genes." retrieved from NCBI Database accession no. U22511 XP002294017
- DA SILVA ET AL.: 'Fastidian gum: the xylella fastidiosa exopolysaccharide possibly involved in bacterial pathogenicity' FEMS. MICROBIOL. LETT. vol. 203, no. 2, September 2001, pages 165 - 171, XP002909501
- LAMBAIS ET AL.: 'A genomic approach to the understanding of xylella fastidiosa pathogenicity' CURR. OPIN. MICROBIOL. vol. 3, no. 5, October 2000, pages 459 - 462, XP002909502

## Description

### FIELD OF THE INVENTION

This invention relates to the isolation of an operon of the microorganism *Xylella fastidiosa.* More particularly, it relates to the isolation of such an operon which comprises nine genes, referred to as "xfgum B, C, D, E, F, H, J, K and M" hereafter. Also a part of the invention are isolated nucleic acid molecules for each of these 9 separate genes. Also a part of the invention are expression vectors which comprise the operon, or one or more of the component genes, in operable linkage with a promoter, recombinant cells which include the operon or one or more of the genes, the expression product of these genes, and various uses thereof.

### BACKGROUND AND PRIOR ART

Xanthan gum is an exopolysaccharide ("EPS" hereafter) produced by the phytopathogenic bacterium *Xanthomonas campestris* (Ielpi et al., 1981, FEBS Lett. 130:253-256). The biosynthesis of this extracellular polysaccharide in *X. campestris* is directed by a cluster of 12 genes, *gumB-gumM* (Becker et al., 1998, Appl Microbiol Biotechnol, 50:145-152; Vojnov et al., 1998, Microbiology 144; 487-493). It is composed of polymerized pentasaccharide repeating units which are assembled by the sequential addition of glucose-1-phosphate, glucose, mannose, glucuronic acid, and mannose on a polyprenol phosphate carrier (Ielpi et al., 1993, J. Bacteriol. 175:2490-2500). The pentasaccharide repeating unit is also O-acetylated and pyruvylated to various degrees (Ielpi et al., 1993, J. Bacteriol. 175:2490-2500).

Transformation with a gene cluster carrying *gumB* and *gumC* restored xanthan production in deficient mutants, suggesting that *gumB* and *gumC* are both involved in the translocation of xanthan across the bacterial membrane (Vojnov et al., 1998, Microbiology 144: 487-493).

Two gene groups are involved in xanthan biosynthesis: the genes *xpsIII, xpsIV* and *xps VI,* which are responsible for synthesis of UDP-glucose, UDP-glucuronic acid and GDP-mannose, respectively (Harding et al., J. Bacteriol 169: 2854-2861 (1987); Harding et al., J. Gen Micobiol 139: 447-457 (1993); Hötte et al., 1999; Köplin et al. J. Bacteriol 174: 191-199 (1992), 1992) and the genes *gum* or *xpsI,* which encode the enzymes involved in the polymerization of the pentasaccharide linked to polyprenol phosphate and secretion of the EPS to the extracellular medium (Ielpi et al., 1992; supra Ielpi et al., 1993 supra).

The *X. campestris gum* genes are grouped in an operon (GUM operon) of about 16 Kb in length, containing 12 genes designated *gumB, C*, *D, E, F, G, H, I, J, K, Land M.* The *gumD* gene encodes the enzyme glucosyltranferase I, responsible for the transference of the glucose-1-phosphate of UDP-glucose to the polyprenol phosphate carrier to form the lipid-monosaccharide. *GumM* encodes the enzyme glucosyltranferase II, which catalyzes the addition of the second glucose residue to form the lipid-disaccharide. *GumH* encodes the enzyme glucosyltranferase III, which catalyzes the addition of the first mannose residue to form the lipid-trisaccharide. *GumK* encodes the enzyme glucosyltranferase IV, which catalyzes the addition of a glucuronic acid residue to form the lipid-tetrasaccharide. *GumI* encodes the enzyme glucosyltranferase V, which catalyzes the addition of a second mannose residue to form the lipid-pentasaccharide. *GumF* encodes the enzyme acetyltransferase I which catalyzes acetylation of the internal mannose residue. *GumG* encodes the enzyme acetyltransferase II which catalyzes the acetylation of the external mannose residue. Finally, gumL encodes the enzyme pyruvate ketal transferase which catalyzes pyruvylation of the external mannose residue. *GumB,* C and *E* are involved in the polymerization and secretion of the EPS through the bacterial membrane while *gumJ* is thought to be involved in a parallel stage of polymerization and secretion of the EPS (Ielpi et al., 1992 supra; Ielpi et al., 1993 supra).

Xanthan has wide commercial application as a viscosifier of aqueous solutions. It is used in both the food and non-food industries and also as a stabilizer of suspensions, emulsions, and foams. Mutant *Xanthomonas* strains defective in the xanthan biosynthesis pathway have been shown to synthesize and polymerize xanthans with variant structures, similar rheological properties, and different viscosities (Hassler and Doherty,1990, Biotechnical Prog 6:182-187). Acetylation and pyruvylation can affect the viscometric properties of xanthan. The presence of pyruvate increases viscosity, whereas acetate decreases viscosity (Hassler and Doherty, supra). The elimination of sugar residues from xanthan side chains also affects viscosity. As compared to wild-type xanthan, polymers lacking the terminal mannose (polytetramers) are poor viscosifiers. In contrast, polymers lacking both the terminal mannose and glucuronic acid residues (polytrimers) are superior viscosifiers (Hassler and Doherty, supra). A nonacetylated and an acetylated tetramer, both lacking the side-chain terminal mannose residue and in the first case lacking an acetate group on an internal mannose residue showed higher viscosity (in the first case) and lower viscosity (in the second case) when compared to a wild-type bacteria (Levy et al.,1996, Biopolymers 38:251-272). A major conformational difference between the higher and lower viscosities is the increased amount of open helical backbone and the increase in the side-chain flexibility high viscosity (Levy et al., 1996, Biopolymers 38:251-272).

Xanthan has also been used as a drug stabilizer, to improve drug absorption, to delay drug release and can also be used in controlled-release formulations (Talukdar et al., 1996, J Pharm Sci 185:537-540). In combination with xanthan gum, alpha-cyclodextrin reduced the first-pass metabolism of morphine in the rectal mucosa and by the liver, and was shown to improve the apparent rectal bioavailability of the opioid about 4 fold (Kondo et al., 1996, Biol Pharm Bull 19:280-286). Xanthan-alginate has been used for encapsulation of enzymes. The xanthan-alginate spheres showed 75% of maximum urease activity even after 20 repeated uses under optimal conditions (Elçin, 1995, Biomaterials 16:1157-1161).

Sustained-release hydrogel suppositories prepared with water-soluble dietary fibers, xanthan gum and locust bean gum have been shown to sustain drug release for a much longer time than commercial suppositories. The mean residence time was higher, without a decrease in the area under the plasma concentration vs. time curve. Histopathological studies showed good biological safety of the hydrogel suppositories to the rectal mucosa. These results suggested that IMC hydrogel suppositories prepared with xanthan gum and locust bean gum were a practical rectal preparation having prolonged action and reduced side effects (Watanabe et al., 1993, Bio Pharm Bull 16:391-394).

The apparent release rate of prednisolone from hydrogels prepared with xanthan decreased with increasing gum concentration, suggestion that the diffusion of drug molecules was mainly controlled by the density of the three-dimensional network structure in the matrix. These results indicated that drug release could be controlled not only by the density of the network structure but also by the microscopic viscosity of the hydrogels (Watanabe et al.,1992, Chem Pharm Bull 40:459-462).

A neomycin-furazolidone-xanthan complex has been shown to increase the antimicrobial activity of the drug (Dumitriu et al., 1993, J. Biomater 7:256-276).

Xanthan gum has also been shown to alleviate diabetes mellitus. Administration of xanthan gum lowered fasting and postload serum glucose and reduced fasting levels of total plasma cholesterol in diabetic subjects. Xanthan gum also tended to lower fasting and postload levels of gastrin and gastric inhibitory polypeptide (GIP) and fasting levels of total and VLDL triglyceride and cholesterol in VLDL and LDL fractions. Subjects reported a sense of fullness after consuming xanthan muffins but no severe digestive symptoms (Osilesi et al., 1985, Am J Clin Nutr 42:597-603).

Several factors have been associated with pathogenicity of phytopathogenic bacteria, including production of extracellular enzymes, production of exopolysaccharides, production of toxins and phytohormones along with factors mediating specific plant pathogen interactions (Agrios, 1988, Plant Pathology, 3rd edition, Academic Press; Alippi, 1992, Agronomie 12:115-122). The role of EPSs in the pathogenicity of several bacteria is well known. They are associated with (i) cell death due to occlusion of xylem vessels, (ii) mucoid lesions and (iii) helping plant/pathogen interaction and bacterial growth in the plant tissues.

Several groups have investigated the role of xanthan in *Xanthomonas* pathogenicity. Although it was believed for a long time that xanthan gum is key to pathogenicity, many authors did not succeed in demonstrating a direct link between this EPS and pathogenicity (Kamoun and Kado, 1990, J. Bacteriol 172:5165-5172). Recently, it has been demonstrated that deletion of *gumD* (Chou et al., 1997, Biochem Biophys Res. Commun 233:265-269) or alterations in the later stages of xanthan biosynthesis (Katzen et al., 1998, J. Bacteriol 180:1607-1617) reduced virulence in causing black rot in broccoli and decreased the aggressiveness of *Xanthomonas* against plants. Although the symptoms of *X. fastidiosa* infection of orange trees are well known, e.g., leaf clorosis, nutritional deficiency symptoms, reduced fruit size, etc. (De Negri,1990, Com. Tec. No. 82, Ext. Rural, Cats, Compinas; Malavolta et al., 1990, Cordeiropolis, v. 11, n. 1, p. 15-18), the mechanism by which the bacteria causes the disease is unknown. Bacterial infection depends on insect vectors such as Oncometopia facialis, Acrogonia terminalis and Dilobopterus costalimai (Gravena, et al., 1997, "Os vetores de Xylella fastidiosa In: Clorose Variegada do Citros"). These insects, when feeding on xylem nutrients, introduce the bacteria into the vessel where bacteria grows fastidiously (Lopes et al., 1996, "Xylella fastidiosa," Fitopathologin Brasileira, Brasilia v. 21, Suplemento p. 343; Roberto et al., 1996, Fitopatologia Brasileira, Brasilia v. 21, n. 4, p. 517-18). The EPS synthesized by the XfGUM operon, which is a feature of the invention, might be directly involved in the pathogenicity causing Citrus Variegated Clorosis in orange trees and other diseases in plants, such as coffee and grape plants.

### SUMMARY OF THE INVENTION

The invention relates, in part, to a nucleic acid molecule of about 12710 nucleotides which encodes the members of the XfGUM operon, including genes for xf*gumB,* xf*gumC,* xf*gumD,* xf*gumE,* xf*gumF,* xf*gumH,* xf*gumJ,* xf*gumK* and xf*gumM* and the uses of such operons, as well as the proteins encoded thereby. These can be used, e.g., to control orange tree diseases, such as Citrus Variegated Clorosis (CVC) and diseases caused by the bacteria in other plants. The EPS produced by the enzymes encoded by the XFGUM operon may cause the diseases by occluding the xylem vessels, or by participating in the mechanism of bacteria infection, adhesion and spreading; however, applicants do not limit themselves to any single theory of operation. The invention also relates to the use of components of the XfGUM operon for production of exopolysaccharides, useful industrial and/or medical application of this new gum and structural gums variants, using *Xylella fastidiosa* or any other bacteria as a host.

The invention is based in a number of unanticipated and surprising discoveries. One is the discovery of a DNA sequence of 12710 nucleotides in the genome of the phytopathogenic bacteria *Xylella fastidiosa.* A xanthan GUM operon has been found previously only in *Xanthomonas campestris.* Another is the fmding that the XfGUM operon lacks genes *gumI, gumG* and *gumL,* which encode the enzymes glucosyltransferase V, acetyltranferase II and pyruvate ketal transferase, respectively. The absence of these genes indicate that the XfGUM operon encodes the enzymes necessary to synthesize an exopolysaccharide composed of tetrameric units consisted of glucose, glucose, mannose and glucuronic acid and that, in view of the absence of the genes encoding the acetyltransferase II and pyruvate ketal transferase, the resulting tetrasaccharide is not pyruvylated at all and, as demonstrated for *Xanthomonas,* its mannose may be only partially acetylated. These findings indicate that XfGUM encodes the enzymes that produce a new gum with new rheological and chemical properties.

The invention includes, inter alia, (a) nucleotide sequences containing the structural and regulatory regions of the XfGUM operon that encode the enzymes glycosyltranferase I, glycosyltranferase II, glycosyltranferase III, glycosyltranferase IV, and acetyltransferase I, as well as the proteins referred to hereafter as GUMB, GUMC, GUME and GUMJ; (b) the expression of such nucleotide sequences in cells; (c) the isolated enzymes, proteins, and active forms thereof, as well as mutants, fragments, and fusion proteins; (d) the uses of the enzymes and/or proteins encoded by the GUM-like Operon in screening methods for identifying compounds that are useful in controlling diseases caused by *Xylella fastidiosa* and/or related organisms; (e) the engineering of bacterial hosts to express the enzymes and/or proteins encoded by XfGUM; and (f) the use of the engineered hosts expressing the enzymes and/or proteins encoded by XfGUM to produce the exopolysaccharide for industrial and/or medical applications, and for animal use, including humans.

### BRIEF DESCRIPTION OF THE FIGURES

Figures 1A-1I compare the deduced amino acid sequences ofthe proteins of the invention with homologous proteins of *Xanthomonas campestris.*
Figure 2 sets forth a proposed biosynthetic pathway using the components of the invention.

### DETAILED DESCRIPTION OF THE PREFERRED EMBODIMENTS

The present invention relates to a nucleic acid molecule of about 12710 nucleotides of the *Xylella fastidiosa* genome that encodes the operon XfGUM comprising genes xf*gumB,* xf*gumC,* xf*gumD,* xf*gumE,* xf*gumF*, xf*gumH*, xf*gumJ,* xf*gumK* and xf*gumM* and the uses of such operon, either as DNA as well as the proteins it encodes, for the control of the orange tree disease Citrus Variegated Clorosis (CVC) or related diseases caused by the bacteria in other plants, such as oak trees and grapevines.

The following definition is provided to remove any potential ambiguities as to the intent or scope of their usage in the specification and claims.

"Similarity" as used herein refers to identity of nucleotide sequences. A sequence is said to be 70% similar to another one if, after a global alignment, 70% of the nucleotides of the smaller sequence are identical to the corresponding nucleotides of the other sequence. Global alignments are done by means of dynamic programming (Thompson JD et al. (1997). Nucleic Acids Res. 25, 4876-4882 incorporated by reference). This guarantees a mathematically optimal alignment assigning a value for matches and mismatches between all nucleotides and penalties for insertions or deletions of different lengths. Typical numbers are a score of 1.9 for a match, 0 for a mismatch, 15 for opening a gap and 6.66 for extending this gap.

### Example 1

A cosmid library was prepared from total DNA of *Xylella fastidiosa,* using standard techniques, including the digestion of the DNA with restriction enzyme BamHI. Those fragments which ranged from about 35-45 kilobases, were selected as being most relevant. A hybridization map was prepared, and reviewed to select the cosmids which did not overlap. Following selection, non-overlapping cosmids were sequenced using the art recognized shotgun approach. One cosmid was selected, in view of similarity to *Xanthomonas spp.* sequences. A fragment of about 12710 nucleotides was isolated, and studied further.

### Example 2

The further studies referred to in example 1, supra, included comparison of the 12710 nucleotide fragment to the known GUM operon of *Xanthomonas campestris.* The known operon has been described as containing 12 genes; however, the operon disclosed herein appears to contain only nine, i.e., it contains genes with some homology to gumB, C, D, E, F, H, J, K and M; however, no sequences homologous to gumG, I, or L were found.

In order to distinguish the two sets of genes, those which are a part of the invention will be described hereafter as "xfgum" genes, and the operon will be referred to as "XfGUM."

The nucleotide sequences ofxfgumB, C, D, E, F, H, J, K and M are set forth as SEQ ID NOS:1-9, respectively. The complete XfGUM operon is set forth at SEQ ID NO:10.

The putative amino acid sequences encoded by the referenced xfgum genes are set forth as SEQ ID NOS:11-19. These were compared to the comparable amino acid sequences for *Xanthomonas campestris.* In figures 1A-1I, homology analysis is presented, using the abbreviations "GUMBXf, GUMCXf," etc., for the proteins of the invention, and "GUMBXc, GUMCXc," etc., for the *X. campestris* proteins.

### Example 3

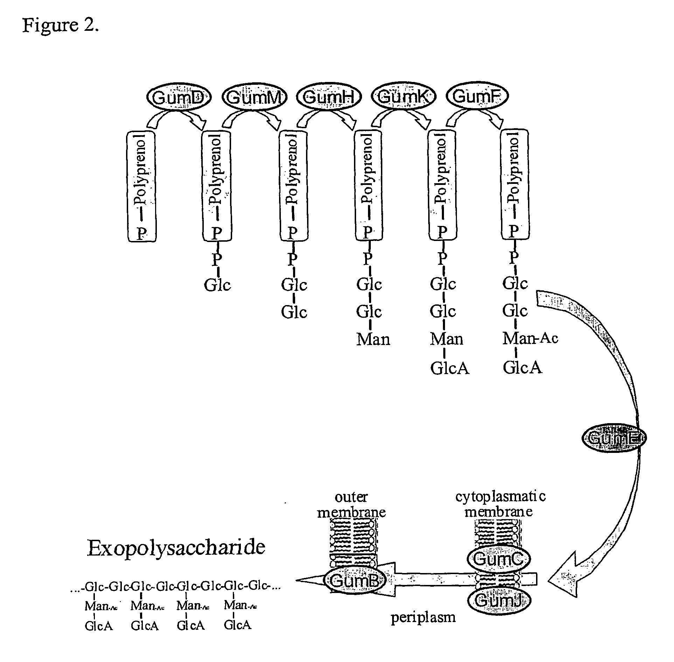

The homology of the sequences from *Xylella fastidiosa* to the *Xanthomonas campestris* sequences led to conclusions about the functions of the encoded proteins, and a proposed mechanism for the synthesis ofthe xanthan-like product by *Xylella fastidiosa,* which is presented in figure 2. Specifically, xfgumD encodes a glycosyltransferase I homologue, involved in addition of a first glucose residue via transfer of glucose-1-phosphate from UDP-glucose to an isoprenol phosphate carrier, resulting in the lipid monosaccharide. The next step in the synthesis involves the addition of a second glucose residue via a glycosyltransferase II homologue, encoded by xfgumM, to form a lipid disaccharide. Following this, a glycosyltransferase III homologue encoded by xfgumH is believed to add a mannose residue to form the trisaccharide, after which a glycosyltransferase IV homologue encoded by xfgumK adds a glucouronic acid residue, forming the tetrasaccharide. The xfgumF gene encodes an acetyltransferase I homologue, which catalyzes acetylation of the mannose reside referred to supra. The proteins encoded by xfgumB, C and E appear to be involved in secretion of the xanthan-like EPS through bacterial membranes, while the protein encoded by xfgumJ appears to be involved in the polymerization and secretion of the xanthan-like EPS.

As noted supra, the XfGUM operon lacks components corresponding to *Xanthomonas spp.* genes GUMG, I and L, which encode acetyltransferase II, glucosyltransferase V, and pyruvate transferase. This suggests that the EPS produced by *Xylella fastidiosa* consists of tetrameric units of glucose-glucose-mannose-glucuronic acid, where these tetramers are not pyruvylated, and are acetylated in whole or part.

Hassler, et al., Biotechnol Proj. 6:182-187 (1990), and Levy, et al., Biopolymers 38:251-272 (1996), both indicate that mutant strains of Xanthanomas, which are defective in one or more steps of the xanthan biosynthetic pathway synthesize structural variants of xanthans, which had chemical properties that differ from each other and standard xanthans. Both acetylation and pyruvylation are known as processes which affect viscometric properties of xanthan. Pyruvate increases viscosity, and acetate decreases it. Further, when terminal mannose is absent, viscosity decreases. Nonacetylated tetramers lacking side chain terminal mannose have higher viscosity. These prior observations suggest that the EPS described herein will have properties that differ from previously identified xanthans.

The foregoing description, including examples, describes features ofthe invention. These include isolated nucleic acid molecules which encode any and all ofthe xfgum proteins described herein, including GUMxfB, C, D, E, F, H, J, K and M, as well as the proteins themselves. Especially preferred are those isolated nucleic acid molecules which comprise or consist of the nucleotide sequences of SEQ ID NOS: 1-9, and nucleic acid molecules which differ therefrom but, in view of the degeneracy of the genetic code, still encode the proteins which have the amino acid sequences set forth in SEQ ID NOS: 11-19.

The isolated nucleic acid molecules of the invention may be included in expression vectors, such that the nucleic acid molecules are in operable linkage with a promoter. In accordance with this feature of the invention, more than one of the nucleic acid molecules described and claimed herein may be included in the expression vector. There are nine nucleic acid molecule families described herein. Any and all combinations of these may be a part of these expression vectors. In other words, combinations of 2, 3, 4, 5, 6, 7, and 8 of the nucleic acid molecules may be a part of these vectors, as well as expression vectors including all 9, or just 1.

Also a part of the invention are recombinant cells, such as recombinant prokaryotic cell strains, and recombinant eukaryotic cell lines, that have been transformed or transfected with one or more of the isolated nucleic acid molecules or recombinant expression vectors of the invention.

Also a part of the invention, as described herein, is the isolated XfGUM operon. A preferred embodiment is that set out in SEQ ID NO: 10; however, it will be understood that the statements regarding the degeneracy of the genetic code referred to supra apply here as well. Further, in connection with the operon, as well as all nucleic acid molecules described herein, principles of preferred codon usage apply in connection with the use of the nucleic acid molecules for transforming or transfecting cells.

As indicated, supra, also a feature of the invention are the proteins encoded by the isolated nucleic acid molecules. These proteins have enzymatic activity, as described herein. Further, they can be used as immunogens to generate antibodies, both polyclonal and monoclonal, which can be used as described infra. Further, these proteins can be produced in various recombinant forms, including fusion proteins, in active forms that consist of less than the entire amino acid sequence of the protein, and so forth. For example, it is well known that enzymes are composed of various structural domains, including one or more active sites. Such active sites are a part of the invention, and comprise less than the entire amino acid sequence of the protein.

As indicated, the proteins of the invention can be used to prepare xanthan like molecules, such as those illustrated in figure 2, i.e.:
[glucose-glucose-mannose-glucuronic acid]n.
where n is a whole number from 1 to about 5000, preferably from about 300 to about 5000, most preferably from about 1500-3000.
This structure may be acetylated; however, as indicated supra, when made via a process that comprises the use of the enzymes that are a part of this invention, the resulting tetrasaccharide structure differs from known xanthans in that it is not completely acetylated or pyruvylated. The compounds described, supra, may be polymerized via intracellular or extracellular processes.

The isolation and characterization of the XFGUM operon allows the skilled artisan to determine what substances act as inhibitors or activators of the operon as a whole, or the individual members of the operon. As indicated supra, the xanthan like products of the invention have various industrial uses, and determination of activators and/or agonists permits the artisan to increase production of the materials under appropriate conditions, such as laboratory culture of *Xylella fastidiosa,* or transformed or transfected cells which produce the xanthan like compound. "In the field" at it were, it is desirable to inhibit or antagonize *X. fastidiosa,* and the operon and isolated nucleic acid molecules of the invention permits the artisan to identify materials which are useful in inhibiting the organism.

Other facets of the invention will be clear to the skilled artisan, and need not be elaborated here.

The terms and expressions which have been employed are used as terms of description and not of limitation, and there is no intention in the use of such terms and expressions of excluding any equivalents of the features shown and described or portions thereof, it being recognized that various modifications are possible within the scope of the invention.

SEQ ID NO: 1

ATGTGTACGGTTATTCTGATGGCTTGCAATACTGCACCGCATTCTCTGGCCTCTTCTCTCCCGGTAC
CTGATGCACTGCTGTTTCCACAAGTGCAGCCTGAATACAAAGTTTCTCCTGGTGACTTATTGTTGAT
CAAGGTATTCCAAGTCGATGATCTGGAGCGTCAGGTCCGTGTGGACAACACTGGGCGTATTACCCTG
CCATTGTTAGGAAGCATTGATGTATCCAATAAAGGTGCGGCTGAGTTGGAAACTCTGATAGCCAATC
GTTACCGCAGTGGCTATCTGCAAAATCCTCAAGTTTCTGTTTTTATCCAAGAAGCGAATGCACGCCG
TGTTACCGTGACTGGTGCAGTGACCGAGCCGGGGAGCTATCCATTGATCGGTAGCAACCTGACATTG
CAGCAAGTGATTGCTCAGGCTAAAGGTATAAACACGCTGGCGAGTCTTCAAAACGTGGTTGTATTCC
GTACTGTCAAAGGACAGAAGATGTTGGCGCGCTTTGATTTGGCACGTATCGAGCGTGGTAAGGATCC
TGATCCTGAGATCTATCCTGGTGATCTCGTTGTTGTTTACCGTTCCGACATGCGCTTGTTTTTGCGC
ACTCTTGTTGAAATTACCCCCTTCGTTATGGTCTGGCGCGCGTATCGATAA

FIGURE 1A

SEQ ID NO: 11 GumBXf MCTVILMACNTAPHSLASSLPVPDALLFPQVQPEYKVSPGDLLLIKVFQVDDLERQVRVD 60
GumBXc ---MSLGACSTGP-EMASSLPHPDPLAMSTVQPEYRLAPGDLLLVKVFQIDDLERQVRID 56
: * **.*.* .:***** **.* :. *****:::******:****:********:*

GumBXf NTGRITLPLLGSIDVSNKGAAELETLIANRYRSGYLQNPQVSVFIQEANARRVTVTGAVT 120
GumBXc QNGHISLPLIGDVKAAGLGVGELEKLVADRYRAGYLQQPQISVFVQESNGRRVTVTGAVD 116
:.*:*:***:*.:..:. *..***.*:*:***:****:**:***:**:*.*********

GumBXf EPGSYPLIGSNLTLQQVIAQAKGINTLASLQNVVVFRTVKGQKMLARFDLARIERGKDPD 180
GumBXc EPGIYPVIGANLTLQQAIAQAKGVSTVASRGNVIVFRMVNGQKMIARFDLTEIEKGANPD 176
*** **:**:******.******:.*:** **:*** *:****:*****:.**:* :**

GumBXf PEIYPGDLVVVYRSDMRLFLRTLVEITPFVMVWRAYR 217
GumBXc PEIYGGDIVVVYRSDARVWLRTMLELTPLVMVWRAYR 213
**** **:******* *::***::*:**:********

SEQ ID NO: 2

ATGGATTATCCCAATCAAACTTCTTCCGCTTTGTTTCACTCCTCAGAACCATTGGGTCTGAATCTTC
TTGATTATTTACGTGTGTTGGTCAGTCAATATTGGCTGATTATTGGTGTTGCCATCGGCGCAACTTC
GCTAGCCCTTGGTGTCTCGTTGCTGCTTCCTCAAAAGTTTCGTGCCACTGCCACGTTACAGATTGAG
CGTGATGTGCCTAAGGTGATGAATGTCGATAATTTGATCCCTGTAGAGTCGCCGCTTGATCGTGATT
TCTATCAAACTCAATATCAGTTATTACAGAGTCGTTCGTTAGCGCGTGCAGTCATTCGTAAGATGAA
CTTGGATCGAGAGCCGATGTTGAAACCACTCGTCGATAAAGTATTGTCAAAGGTACAAAATCTTCCA
GTTAATACTCGTAACACAGCTATCGAGAGTGCATTGATAGAAATGATGTTGAACAGTTTAAAGATCG
AACCGATCCTCAATTCGAGGCTGGTCTATGTGCATTTTGATTCTCATGATCCCACCTTATCGGCGAA
GGTTGCTAATGCCTACGCCAAGATGTTCATTGAAAATAATCAGCAGCGTCGTTCAAATGCCTTCTCA
TTTGCAATGAAGTATCTTGCTGGACGCTTAGAACAATTGCGTATCAAGGTGGATAAGTCAGAAAGGA
ATGTGGTTGCGTATTCCACTGATGAAAAGATTGTTTCTGTGGGTGACGAAAAACCATCACTCTCTGC
ACAGAACCTGAGTGATCTTAATGCGTTGCTGGCCTCCGCACAGAATGAGCGGATTAGGGCCGAAGCC
TCTTGGCGTCAGGCCAGTATTGGTGATGGTCTGAGTATCCCTCAAGTGTTGTCTAACCTGTTGGTAC
AGTCTTTGCGTACCGAGCAGGCGAATATGGTCAATGAATATCAGCAGAAATTATCCATGTTTAAACC
TGAGTATCCGGAGATGCAACGGTTGAAAGCAAGAATCAAAGAAAATACTAAGCAAATCAATGCTGAG
GTATTGAATATTAGGCAATCGTTGAAGTCTCAGTATGAAGCTACATTACGTCAGGAGAATCTATTGA
ATGACCGTATTGCAGTGTTGGAGAAGGATGAGTTGGATCTGAAAACGCGTCTTATTCGTTATAACCT
ACTCGAGCGTGAGGCTGAAACAGATCGCCAACTTTATGATGCGTTGTTGCAACGTTATAAGGAAATT
AGTGTGCTTGGCGATGTTGGTAGTAACAATGTGACGGTAGTGGATGCGGCAGATATTCCGAGCCGCC
CAATTTCGCCGAATTTGTTAATGAACACGATATTGGGTGGAATTTTTGGTGTCTTTTTGGGTTTGAC
GGTTGCGATGGTGCGTTATTCCATGCATGGAGCGAAGCGGATAAACGTGATGAGCGTGGAATAA

FIGURE 1B

SEQ ID NO: 12 GumCXf MDYPNQTSSALFHSSEPLGLNLLDYLRVLVSQYWLIIGVAIGATSLALGVSLLLPQKFRA 60
GumCXc ----------------------MDYWRALVSQLWLIILIAVGALLLAFGITMLMPEKYRA 38
:** *.**** **** :*:** **:*:::*:*:*:**

GumCXf TATLQIERDVPKVMNVDNLIPVESPLDRDFYQTQYQLLQSRSLARAVIRKMNLDREPMLK 120
GumCXc TSTLQIERDSLNVVNVDNLMPVESPQDRDFYQTQYQLLQSRSLARAVIREAKLDQEPAFK 98
*:*******  :*:*****:***** ***********************: :**:** :*

GumCXf PLVDKVLSKVQN-LP-----VNTRNTAIESALIEMMLNSLKIEPILNSRLVYVHFDSHDP 174
GumCXc EQVEEALAKAAEKNPEAGKSLDSRQAIVERSLTDTLLAGLVVEPILNSRLVYVNYDSPDP 158
*::.*:*. :  *     :::*:: :* :* : :* .* :***********::** **

GumCXf TLSAKVANAYAKMFIENNQQRRSNAFSFAMKYLAGRLEQLRIKVDKSERNVVAYSTDEKI 234
GumCXc VLAAKIANTYPKVFIVSTQERRMKASSFATQFLAERLKQLREKVEDSEKDLVSYSTEEQI 218
.*:**:**:*.*:** ..*:** :* *** ::** **:*** **:.**:::*:***:*:*

GumCXf VSVGDEKPSLSAQNLSDLNALLASAQNERIRAEASWRQASIGDGLSIPQVLSNLLVQSLR 294
GumCXc VSVGDDKPSLPAQNLTDLNALLASAQDARIKAESAWRQASSGDGMSLPQVLSSPLIQSLR 278
*****:****.****:**********: **:**::***** ***:*:*****. *:****

GumCXf TEQANMVNEYQQKLSMFKPEYPEMQRLKARIKENTKQINAEVLNIRQSLKSQYEATLRQE 354
GumCXc SEQVRLTSEYQQKLSTFKPDYPEMQRLKAQIEESRRQINGEVINIRQSLKATYDASVHQE 338
:**..:..******* ***:*********:*:*. :***.**:*******: *:*:::**

GumCXf NLLNDRIAVLEKDELDLKTRLIRYNLLEREAETDRQLYDALLQRYKEISVLGDVGSNNVT 414
GumCXc QLLNDRIAGLRSNELDLQSRSIRYNMLKRDVDTNRQLYDALLQRYKEIGVASNVGANNVT 398
:******* *..:****::* ****:*:*:.:*:**************.* .:**:****

GumCXf VVDAADIPSRPISPNLLMNTILGGIFGVFLGLTVAMVRYSMHGAKRINVMSVE 467
GumCXc IVDTADVPTSKTSPKLKLNLALGLIFGVFLGVAVALVRYFLRGPSPRSRLN-- 449
:**:**:*:   **:* :*  ** *******::**:*** ::*..  . :.

SEQ ID NO. 3

ATGCTTCTGGCAGATCTGAACAGTGTTACTGATATTGCTTCTTCACCGCGGTTATTGTCGAAATATT
CTGCTGCCGCTGATCTAATACTTCGTGTTTTGATCTAACAATGGTGGTGCTCTCTGGTGTAATGAT
TTACAGATTGTTGTTTGGTACTTGGATGTTAATGGTCCCATATCGTATTGCGATTGGTAACACGTTA
CTGTATTCGGTGATTTGTTTTTCTTTTTTCCCGTTGTATCGCAGTTGGCGTGGGCGTGCTCTGGCGC
GGGAGCTGTTAGTACTTAGTAGTGCGTTCTGTGGTGTATTTATCTTGTTTTCGTTGCATACACTGAT
TGTTCAATTGGGACATCCTGTCTCCTGGTTATGGATTACTTTCTGGTTCCTTGGTGCGCTCTCTACC
TTGTTGAGTGCGCGTGTAGCGCTTCGTAGTCTACTCAACTGGTTGCATATGCGTGGTGTGGATGTCC
AACGTATTGTTGTGGTTGGGCTGCGTCATCCTGTGATGAAAATTCACCATTATCTGAATCGTAATAC
ATGGACAGGGATGCAATTGATTGGGTATTTCAGTACTTCCTACGATGTCTCTGTATCTGAATATGTG
AGGCGTCTTCCGTCTCTTGGACGCCGGATAGATTGTTCGATTTTTAGAAAAAAATCACGTTGAGC
AAGTATGGATTTCGATGCCTCTTGGTGAGCGTGATTATATTAAGACGTTGTTGAAGAAATTGGAGCG
TTATCCGATCAATGTAAAACTTATTCCTGATTTATTCGACTTTGGCACACTCAATCAGTCGGGTGAA
CAAATTGGTCATGTGCCTGTCATTAATCTTCGTCAGGGCGGTGTTGATCGAGATAACTACTTTTTTG
TTGCCAAGACAATCCAGGATAAAGTGCTGGCCATGATTGCATTGCTGTTGCTGTGGCCGTTGTTAAT
CATCATTGGGATTGGTATCAAGTTCAGTTCCCCAGGTCCGGTATTGTTCCGGCAACGCCGCCATGGC
TTGAATGGGCGTGAGTTTTATGTGCTCAAATTTCGTTCGATGCGGGTGCATGACGATCAAAGTCAGC
ATCTGAAGCAAGCCAGTCGTAATGACAGTCGTATCACTGCTTTTGGTGCGTTCCTGCGTCGCACCAG
TCTGGATGAGTTACCACAAATCTTCAATGTACTTGGCGGCAGTATGTCGATTGTTGGCCCGCGTCCT
CATGCGGCGCAGCACAACAGTTATTACGAGAAATTGATCCAGCATTACATGCAACGCCATTACGTCA
AACCAGGGATTACTGGGTGGCGCAGGTCAACGGTTTTCGTGGTGAAACACAAGAATTAAGGACAAT
GAAAAAGCGTATTCAGTACGATCTTGATTACATCCGGCGCTGGTCATTGTGGTTCGATTTTCGAATT
ATTGTTTTGACTTTAGTACGTGTACTTGGTCAGAAAAATGCTTATTGA

FIGURE 1C

SEQ ID NO: 13
GumDXf MLLADLNSVTDIASSPRLLSKYSAAADLILRVFDLTMVVLSGVMIYRLLFGTWMLMVPYR 60
GumDXc MLLADLSSATYTTSSPRLLSKYSAAADLVLRVFDLTMVVASGLIAYRIVFGTWVPAAPYR 60
******.*.*  :***************:**********  **::  **::****:   .***

GumDXf IAIGNTLLYSVICFSFFPLYRSWRGRALARELLVLSSAFCGVFILFSLHTLIVQLGHPVS 120
GumDXc VAIATTLLYSVICFALFPLYRSWRGRGLLSELVVLGGAFGGVFALFAVHALIVQVGEQVS 120
:**..*********::**********.*   **:**..** *** **::*:****:*. **

GumDXf WLWITFWFLGALSTLLSARVALRSLLNWLHMRGVDVQRIVVVGLRHPVMKIHHYLNRNTW 180
GumDXc RGWVGLWFVGGLVSLVAARTLLRGFLNHLRTQGVDVQRVVVVGLRHPVMKISHYLSRNPW 180
*:  :**:*.*  :*::**.  **.:** *:  :*****:************ ***.**.*

GumDXf TGMQLIGYFSTSYDVSVSEYVRRLPSLGTPDRLFDFLEKNHVEQVWISMPLGERDYIKTL 240
GumDXc VGMNMVGYFRTPYDLAVAEQRQGLPCLGDPDELIEYLKNNQVEQVWISLPLGERDHIKQL 240
.**:::*** *.**::*:*    : **.** **.*:::*::*******:******:** *

GumDXf LKKLERYPINVKLIPDLFDFGTLNQSGEQIGHVPVINLRQGGVDRDNYFFVAKTIQDKVL 300
GumDXc LQRLDRYPINVKLVPDLFDFGLLNQSAEQIGSVPVINLRQGGVDRDNYFVVAKALQDKIL 300
*::*:********:******* ****.**** *****************.****::***:*

GumDXf AMIALLLLWPLLIIIGIGIKFSSPGPVLFRQRRHGLNGREFYVLKFRSMRVHDDQSQHLK 360
GumDXc AVIALMGLWPLMLAIAVGVKMSSPGPVFFRQRRHGLGGREFYMFKFRSMRVHDDHGTTIQ 360
*:***:  ****::  *.:*:*:******:********.*****::**********:.   ::

GumDXf QASRNDSRITAFGAFLRRTSLDELPQIFNVLGGSMSIVGPRPHAAQHNSYYEKLIQHYMQ 420
GumDXc QATKNDTRITRFGSFLRRSSLDELPQIFNVLGGSMSIVGPRPHAAQHNTHYEKLINHYMQ 420
**::**:*** **:****:*********************************::*****:****

GumDXf RHYVKPGITGWAQVNGFRGETQELRTMKKRIQYDLDYIRRWSLWFDFRIIVLTLVRVLGQ 480
GumDXc RHYVKPGITGWAQVNGFRGETPELRTMKKRIQYDLDYIRRWSLWLDIRIIVLTAVRVLGQ 480
********************* ***********************:*:****** ******

GumDXf KNAY 484
GumDXc KTAY 484
*.**

SEQ ID NO: 4

ATGTTTCAGTCCATGCATGGCTGTGTCAGGAGAGTTTCCATGCTGATGCGTATGAGAAAGATCACCA
AAGAGCATTCACGGAATCTTCTTGTTGGAATAGTGCTGTTTTTGGAGTGGCTTATAACTTTCCGTT
AGCCATGATTAACGCCAAAGTTTTTACGGTGACTCCAGTGATGACTTATGCAGTGGAGTTGGTAATT
TATTTTGCCTGCTTCATACTCGGATTATCTGCATTGGATCGCAAGCGCGTTGTGCTTGTGATGAGTG
GTCTTGGGTTTCTCGTTGCATTGACGTTGCTACGGTTTCTGATGAGTTTGGATTTTGATCCAAAATT
CTTCAGGGATGCGCTGATTCCATTCGCTTTTCTGATACTCGGTGCAGCCTACCGTGGATCTTTGCTG
CGTCTATTCATGGGCATGAGTATTTTTATTACTTTTGTTGCTGTTTTCGAGTTGATGATGCCTGATG
TTTATGGTGACATTGCCAATCCGAAAAGTTATTTCGTGAATTCTCGTGGTGCTAGCGCAGACGGTTT
CTGGAATGAAGGGAGTAGTTTGTATCTTAGTGCCACGCGTCCGGATGAGCGTAACTTCTTTGCTGGT
TCGAATTTACCACGTGCATCTTCTGTGTTCATTGAACCGGTGACGATGGGAAATTACATTATCTTTT
TTGCTGCAATTGTGTTGGTTTTCTGGCGTTGGATGAGTGTTTCCGAGTTAATTTTATCGATCGCAAT
GATCTTATTTTTAATTGTCGCTTCAGATGGCCGTTTGGCGACTGGTACATGCATATTAATGGTATTG
CTTGCACCATTTCTTAAACGTTTTGATCAGCGTTTTGCGTTTTTGTTATTTTTTTCGGTGTTACTCG
GTGGATGGTTCATAGTTCAGGTGAATGGTATTCATGCATATGACAAGGATACGATTCTGAGCAGGAC
TTTTTTCAGTGTGTATTCACTGGCTCACTTGCCCTTGGATTCGTGGTTTGGCTTGGATGTTCAGTCG
TCCTATCGTTATTTTGATAGTGGTATTGCTTATTTCATCGCGTCACAGTCAGTTATCACTGTACTTG
TTTTTTTGTTGGCTTATTCATTTCTTTTCGACATGCAAAGTCATGAGGGGCGGGATTTAAAAACAT
GGCCATATTCGCATTTGCATTGAGTCTTTTGGTATCAAATAGTTATTTTTCGATTAAAACGTCTGCG
TTGTGGTGGTTTACGTGTGGCTGTCTTTGGAATTGCTCGCCTAAGGTATTGGCTGCAAAAAGAATC
TTGTAGAAGCGAGAGTGATAGCACAAGGAGGTGTGTCGTGA

FIGURE 1D

SEQ ID NO: 14
GumEXf MFQSMHGCVRRVSMLMRMRKITKEHSRNLLVGIVLFFGVAYNFPLAMINAKVFTVTPVMT 60
GumEXc -------------MLIQMSEQARVRWHNALIELTLLTGVGYNLLLALINANVFTVRPVIT 47
**:::* : :: : :* *: :.*: **.**: **:***:**** **:*

GumEXf YAVELVIYFACFILGLSALDRKRVVLVMSGLGFLVALTLLRFLMSLDFDPKFFRDALIPF 120
GumEXc YAVEFLVYAACFLLGLGSMSRQRIAMIFGGLGLIVTLMFVRFLVNWQIDPKFFRDALVVF 107
****:::* ***:***.::.*:*:.:::.***::*:* ::***:. ::*********: *

GumEXf AFLILGAAYRGSLLRLFMGMSIFITFVAVFELMMPDVYGDIANPKSYFVNSRGASADGFW 180
GumEXc AFVVLGSAYTGSLPKLFIRMTIIVSLVAAFELAMPSAYGDLVNPKSFFVNARGMSAEGFW 167
**::**:** *** :**: *:*:::.**.*** **..***:.****:***:** **:***

GumEXf NEGSSLYLSATRPDERNFFAGSNLPRASSVFIEPVTMGNYIIFFAAIVLVFWRWMSVSEL 240
GumEXc NEDSNLFVSATRPGERNFLPGSNLPRASSWFIEPVTMGNYICFFTAIVLTFWRWMRPSML 227
**.*.*::*****.****:.********* ***********  **:****.*****  * *

GumEXf ILSIAMILFLIVASDGRLATGTCILMVLLAPFLKRFDQRFAFLLFFSVLLGGWFIVQVNG 300
GumEXc ILSIGLIGFMIVASDGRLAAGTCVLMVLLSPLLKRMDQRLAFLLFLFVIASAWLLVWMTG 287
****.:* *:*********:***:*****:*:***:***:*****: *: ..*::* :.*

GumEXf IHAYDKDTILSRTFFSVYSLAHLPLDSWFGLDVQSSYRYFDSGIAYFIASQSVITVLVFL 360
GumEXc ITAY-QDTTMGRIFFTVNSMNNLSFESWMGLDFAQAYRYFDSGISYFIASQSIVGVLAFL 346
* ** :** :.* **:* *: :*.::**:***. .:********:*******:: **.**

GumEXf LAYSFLFDMQSHEGRGFKNMAIFAFALSLLVSNSYFSIKTSALWWFTCGCLWNCSPKVLA 420
GumEXc LSYSFLLLMPSKEGQLFKNQAMFAFALSLLVSNGYFSIKTSALWWFVCGCMWHLMPAASA 406
*:****: * *:**: *** *:***********.************.***:*: * . *

GumEXf AKKNLVEARVIAQGGVS--------- 437
GumEXc VPVRDESKEDPTDNGVHVPLPAGAPR 432
. . . . ::.**

SEQ ID NO:5

GTGATTCATGAAAAAAAGTATGTGCCAGTACGAGGAGGGACATTAGCACACTCTCCGGATCTTTAT
CAGTTGGGCGTGATGCACGTATTGATGCGGCGAAGGACTAGGTATTTTTCTGGTAGTACTTGGTCA
TGCTAAAGGGCTTCCAGAATGGTGGGTTGTGCTGATTTACAGTTTCCACGTGCCGTTGTTTTTCTG
TTATCTGGTTGGGCTGCTGGCACGAATATGCGTCAGTCTGGATTCTGCGAATGTTTGTTGCATTTGA
TGCGTACCTTACTGGTTCCATATGTATTCTTTTTTTCGTTGGTTATATCTATTGGTTGCTTACCCG
TCATATTGGTGCGAAGGATTTGCGTTGGGGGATGCACCCTTGGTGGGAGCCGTTGAATGGTTTGTTG
ATGGGGGGAGGAACTGCTTTGTATGTTCATCCGTCGTTGTGGTTTCTTCCAGCATTGTTTGTGACGA
AATTAACCTATCAAATGTTGTCTAAGTATGTTTCTTTAGAACGATTGGTGTTGTTTGGTGGATATT
TTCTTGGGTATGGGTAGGGTTTTTCCTGGGTTTGGTGTGCGTTTCCCATTTGCTTTAGATGAATTG
CCTATTGCATTTTTATTTTTCGTTTTTGGAGTGATGGGACGCAGAACGTCTTGGTTACGGTTGCTCC
CGAAAACAAGAAAAGCGAATATGATTTACTTGCCGTGTTGTTGTTTCCTTGGTTTCTGTTGGCGTT
GTGCAATGAAAAAGTTGATATGAATATGCTTATTTTTGGCAATTCACCATTTGTTTTTCATGTTTCC
GCATTGTTAGGTGTGGCGATAGTGTTATGTGCGGCGGCTCTTGTTGAGCAATGGTCATTGGTACAAT
GGGCTGGGCGTAATACGCTGTTGATTTTGTGCACGCATACGCTAGTTTTTTTGTGTTATTCGGAGT
ATTGTCTTTGCTCGGAGGACATCTGGATTGATCTTGGCATTGCTTTTTCAGCAATTACGTTGTGT
TTTATTCCAATTTTTAGATGGATATTGATGAGGTGGATACCTTGGTCACTGGGTGCATGTTCATATA
TGCGATCGCGTTCATCATGA

FIGURE 1E

SEQ ID NO: 15 GumFXf ----MIHEKKYVPVRG-GTLAHSPGSLSVGRDARIDAAKGLGIFLVVLGHAKGLPEWWVV 55
GumFXc MNTVTGASGTSAPVQAAGARAFASG---RSRDPRIDATKAIAILLVVFCHAKGVPHGMTL 57
. . .**:. *: *.:.* .**.****:*.:.*:***: ****:*. .:

GumFXf LIYSFHVPLFFLLSGW-AAGTNMRQSGFCECLLHLMRTLLVPYVFFFFVGYIYWLLTRHI 114
GumFXc FAYSFHVPLFFLVSGWLAAGYASRTTSLLQTITKQARGLLLPYVVFYLLGYVYWLLTRNI 117
: **********:*** *** * :.: : : : * **:***.*:::**:******:*

GumFXf GAKDLRWGMHPWWEPLNGLLMGGGTALYVHPSLWFLPALFVTKLTYQMLSKYVSLERLVL 174
GumFXc GEKAARWGSHPWWEPIVSMFTGVGPDLYVQPPLWFLPVMLVTVIGYVLLRRWMPPLVIAA 177
* * *** ******: .:: * *. ***:*.*****.::** : * .:* :::. :.

GumFXf FGGIFSWVWVGFFPGFGVRFPFALDELPIAFLFFVFGVMGRRTSWLRLLPKTRKANMILL 234
GumFXc VAVVLAWFWMNWFPLQHMRLFWGLDVLPVSLCFYALGALLIHVS--PYLPTSLPGSALVT 235
.. :::*.*:.:** :*: :.** **::: *:.:*.: :.* **.: .. ::

GumFXf AVL--LFPWFLLALCNEKVDMNMLIFGNSPFVFHVSALLGVAIVLCAAALVEQWSLVQWA 292
GumFXc VVLAALVAW--LAGVNGRIDVNMLEFGRQHAVFLLSAVAGSLMVICAARMVQEWTWLQWI 293
.** *..* ** * ::*:*** **.. ** :**: * :*:*** :*::*: :**

GumFXf GRNTLLILCTHTLVFFVLFGVLSLLGGTS----GLILALLFSAITLCF-IPIFRWILMRW 347
GumFXc GRNTLLILCTHMLVFFVLSGVAALAGGFGGARPGLGWAIFVTLFALVASVP-LRWFLMRF 352
*********** ****** ** :* ** . ** *::.: ::* :* :**:***:

GumFXf IPWSLGACSYMRSRSS 363
GumFXc APWTLGA----RPVSA 364
**:*** *. *:

SEQ ID NO:6

ATGGAATGGGAGCACTGTTTAATGAAAGTGGTGCATGTTGTTCGTCAATTTCATCCGTCAATTGGTG
GGATGGAGGATGTGGTCTTCAATATTGCGATGCAGCTGCATTTGCATGCTGGAATCGATGTTGATGT
CGTGACGTTGAACCGAGTATTTACTCAGTCTGATGTTCTACTTCCATGTACTGATAAGTATCAGGGT
GTGTCGATCCAGCGCATTGGTTATCGTGGTTCTTCGCGTTATCCATTGGCACCATGGGTATTGCGTA
TGTTGGATAAAGCCGATGTGATTCATGTGCACGGTATTGATTTTTTTATGACTTTCTGGCATTGAC
TCGGGTGTTACATGGAAAGCCAATGGTCGTTTCAACGCACGGTGGTTTCTTCCATACGGATTATGCA
TCAAGACTTAAATTACTTTGGTTCAATACGTTGACTAGGTTATCAGCGTTGGCTTATGCAAGGATTA
TTGCGAGTAGTGAGAGTGATGGTGCGTTGTTTTCAAAGATCGTGGCACCGAGTCGATTGCGAGTCAT
TGAAAATGGCGTAGATGTGGAGAAGTATGCAAGGTGTGGTGCTTCGGAAGCTGGACGCACTCTTTTG
TATTTCGGGCGCTGGTCGATGAACAAGGGATTGTTGGAGACGTTGCAATTACTCGCTGTATTGTATG
TGTTAGATCCACGCTGGCGATTAATTATTGCTGGACGTGAATATGATTATGATCAGGCTGCGTTGGC
GTATGAGGTGGATAGGCTTGGGTTGTCTGAGCAGGTGCATTTTCATTGTAGTCCGTCACAGTCGCAG
TTGCGCTTTTTAATGGAGCAAGCACAGTTTTTATTTCACTGTCGCGGCATGAGGGTTTTGGTATCG
CTGCAGTTGAGGCGATGAGTGCTGGATTAATTCCTGTCTTGAGTGATATTCCACCATTTGCGCGTCT
ACATCGTGAGTCCGGTTTAGGTGTTTTGGTCGATCCATTACAACCACAGCAGGCTGCAGTTGCTGTA
CAGGGATTGGCAGTACAGGTAGATACTCATTTTATCGACTGGCGATCTCAAGCGATGGCTTTCAGTG
ATCGTTATCACTGGCGTTATGTCATTGGTTGTTATCAGGATGAATATTGTAGAGCGCTTGGATTAGG
TGGCGAGCAGGAATTTCTGAGGTGA

FIGURE 1F

SEQ ID NO: 16
GumHXf     MEWEHCLMKVVHVVRQFHPSIGGMEDVVFNIAMQLHLHAGIDVDVVTLNRVFTQSDVLLP 60
GumHXc     -------MKVVHVVRQFHPSIGGMEEVVLNVARQHQANSADTVEIVTLDRVFTDPSAQLA 53
                  ******************:**:*:* *  :  ::.   *::***:****:... *.

GumHXf     CTDKYQGVSIQRIGYRGSSRYPLAPWVLRMLDKADVIHVHGIDFFYDFLALTRVLHGKPM 120
GumHXc     QHELHQGLSITRIGYRGSSRYPIAPSVLGAIRSADVVHLHGIDFFYDYLALTKPLHGKPM 113
             : :**:** ***********:** **  : .***:*:********:****: ******

GumHXf     VVSTHGGFFHTDYASRLKLLWFNTLTRLSALAYARIIASSESDGALFSKIVAPSRLRVIE 180
GumHXc     VVSTHGGFFHTAYASRMKQIWFQTLTRTSALAYARVIATSENDGDLFAKVVAPSRLRVIE 173
           *********** ****:* :**:**** *******:**:**.** **:*:**********

GumHXf     NGVDVEKYARCGASEAGRTLLYFGRWSMNKGLLETLQLLAVLYVLDPRWRLIIAGREYDY 240
GumHXc     NGVDVEKYAGQGARAPGRTMLYFGRWSVNKGLIETLELLQAALTRDPQWRLIIAGREYDL 233
           *********  **  .***:*******:****:***:** .  . **:**********

GumHXf     DQAALAYEVDRLGLSEQVHFHCSPSQSQLRFLMEQAQFFISLSRHEGFGIAAVEAMSAGL 300
GumHXc     NEADLRKAIAERGLQDKVQLSMSPSQQQLCALMQQAQFFVCLSRHEGFGIAAVEAMSAGL 293
           ::* *   : . **.::*::  ****.**  **:*****:.******************

GumHXf     IPVLSDIPPFARLHRESGLGVLVDPLQPQQAAVAVQGLAVQVDTHFIDWRSQAMAFSDRY 360
GumHXc     IPILSDIPPFVRLATESGQGVIVNRDRIQAAADSVQALALQANADFDARRTATMAYVARY 353
           **:*******.**  *** **:*:  :.* ** :**.**:*.::.*   *: :**:  **

GumHXf     HWRYVIGCYQDEYCRALGLGGEQEFLR 387
GumHXc     DWRHVVGRYIDEYHAALGTPRTQEAVR 380
           .**:*:* * *** ***   ** :*

SEQ ID NO: 7
ATGAGTGACAGTGTGGTGAGTGTTTCTGCGGTGTCCGAGCGTAGTTTGGCTTCACGTGCGCTTGGCG
GTGCCGTGGTGACGATGCTTGGGCAGGGGCGAGGGTAGTTATCCAATTTTCCATTATCGTGCTATT
GGCGCGGTTGTTGAATCCGCATGATTATGGTCTGATGGCGATGGTCACTGCGATTGTGGGTGTTGCT
GATATTCTTCGTGATTTTGGCCTTTCTTCGGCGGCGATCCAGGCCAAGCAGATTACAAACGCGCAGC
GCGATAATCTATTCTGGATTAATAGTGTGATCGGATTGGCACTGTCGTTGGTGGTATTCGTTGCGGC
ACAGCTAATTGCTGATTTTTATCGTGAACCTGCATTAGTGACGATTACGCAGGTATTGGCGATTAAT
TTTTTACTTAATGGATGGCAACTCAGTATCGCGCCAATCTTAGCCGTGAGATGCGTTTCGGTCAGC
TTGCATTGAGTGATATTGGTGCGCAGGTGTTAGGTCTGTTAGTTGGTGTTAGTGTGGCGCTGGTCGG
ATGGGGCGTTTGGGCGTTGGTGTTGCAGCAGGTGGTGCAAGCTGTGGCGAATCTGGTAATTGCAATG
GTCTGTGCGCGTTGGCTGCCAGGTGGTTATCGCCGTGGTGTGCCGATGGGGAGTTTTCTCAGTTTTG
GTTGGAATCTGATGGTTGCGCAATTGCTAAGCTATGCCAATCGCAGTGTTGGTCAAGTGATTATCGG
TTATCGGCTTGGTCCTAATGTACTTGGTTTGTATAACCGTGCATTCCAGTTGTTGATGATGCCATTG
AATCAGGTGATTGCACCGGCAAGTTCAGTGGCGTTGCCGGTGCTTTCTCAGTTGCAAGATGATCGTG
CTCGTTTCGATAGCTTCTTATTGCGTGGCAAACGATGATGTTGCATGTGATTGTTGCGTTGTTTGC
ATTTTCTTGTGCGCAGGCCACTCCATTAATTGTATTGGTCCTTGGTGAAAAGTGGCGTTCGGCGGTG
TTGTTATTCCAAATTCTGACATTGGCTGGTATGACGCAGAGTGCGAGTTATGCAAGTTATTGGCTAT
TTTTAGCACGCGGTTTGATACGCGATCATCTTTTATTTTCTATTGTCAGTCATGTGTTTTTGGTGTT
GTGTGTGTGCATTGGTGCTTATTGGGGTGTATTTGGTGTCGCTATTGGTTACAGCATTAGTTTAGCT
TTGATATGGCCATTGTCGATTATTTGGGCGGCGCGTATTACACCTGTTCCTGGTTGGAAATGTTTT
TCAATGGGATGCGTGCGATCGTAGGTTACGGGGTGTGTGCATTCGCTTCTATGTATGCTTCGCAGTG
GTGTGACGAGTCGAATCTTTGGAAGCAATTGATAGTTGGTGCTTTGGCAATGTTGGTAGCTTTTGCA
GTGTTATCCTTGTTATGGTCAGCATTTCGTCGTGATGTGCTGTCTATCATTAAGATCGGTATGTCTT
CTTCAGTAGTATCTTCTTTTCTTCTAAGGATCATGAGGAGAGTGAGGAAGGATCTTAA

FIGURE 1G
SEQ ID NO: 17 GumJXf MSDSVVSVSAVSE--RSLASRALGGAVVTMLGQGARVVIQFSIIVLLARLLNPHDYGLMA 58
GumJXc MTAETSTMTSPTPPPRSLGSRAAG-AAVTMIGQSAKMIVQFGGIVLLARLLTPYDYGLMA 59
*: .. :::: : ***.*** * *.***:**.*::::**. ********.*:******

GumJXf MVTAIVGVADILRDFGLSSAAIQAKQITNAQRDNLFWINSVIGLALSLVVFVAAQLIADF 118
GumJXc MVTAIVGAAEILRDFGLSAAAVQAKHVSREQRDNLFWINSGIGLMLSVVVFASAHWIADF 119
*******.*:********:**:***::::. ********** *** **:***.:*: ****

GumJXf YREPALVTITQVLAINFLLNGMATQYPANLSREMRFGQLALSDIGAQVLGLLVGVSVALV 178
GumJXc YHEPALVTISQALAVTFLLNGMTTQYRAHLSRGLRFGQVALSDVGSQVLGLGAAVAAALA 179
*:*******:*.**:.******:****:*** :****:****:*:***** ..*:.**.

GumJXf GWGVWALVLQQVVQAVANLVIAMVCARWLPGGYRRGVPMGSFLSFGWNLMVAQLLSYANR 238
GumJXc GWGYWALIVQQVVQAIVNLIIAGACARWLPRGYARQAPMRDFMSFGWNLMAAQLLGYASR 239
*** ***::******:.**:** .****** ** * .** .*:*******.****.**.*

GumJXf SVGQVIIGYRLGPNVLGLYNRAFQLLMMPLNQVIAPASSVALPVLSQLQDDRARFDSFLL 298
GumJXc NVGQVIIGWRTGPDALGLYNRAFQLLMMPLNQINAPATSVALPVLSQLQDERERYSAFLL 299
.*******:* **:.****************: ***:*************:* *:.:***

GumJXf RGQTMMLHVIVALFAFSCAQATPLIVLVLGEKWRSAVLLFQILTLAGMTQSASYASYWLF 358
GumJXc RGQTVMVHLIFALFAFACALAMPLIVLVLGEQWREAVPLFQVLTLGGIFQTASYATYWVF 359
****:*:*:*.*****:** * *********:**.** ***:***.*: *:****:**:*

GumJXf LARGLIRDHLLFSIVSHVFLVLCVCIGAYWGVFGVAIGYSISLALIWPLSIIWAARITPV 418
GumJXc LSKGLMREQLVYSLVGRILLIACIFVGSRWGAMGVAIGYSFGLLLIWPLSLVWIGKITDA 419
*::**:*::*::*:*.:::*: *: :*: **.:*******:.* ******::* .:** .

GumJXf PGWEMFFNGMRAIVGYGVCAFASMYASQWCDESNLWKQLIVGALAMLVAFAVLSLLWSAF 478
GumJXc PVGALFVNAMRALVAYGIAGGCAYYASVTVG-GPLWQQLLVGAGAMALVC-LLALAWPGF 477
* :*.*.***:*.**:.. .: *** . . **:**:*** ** :. :*:* *..*

GumJXf RRDVLSIIKIGMSSSVVSSFLLRIMRPVRKGS 510
GumJXc RRDVVAIVNIRK-------LLTQAKAPP---- 498
****::*::* :* : *

SEQ ID NO: 8
GTGGTTGAGTATAATGGTGTGCATTGTTATCTATGGCGTACGTTGGTACATCCGTTTAATACACGTA
GATCATGGTTGCGTGGTATTGAGGATATGCTCTTTCGCTGGTATGTGCAGCATCCACCGGAGATTTT
ATTGCAGTGGATACGCGAAGCAGACATCATTTTGTTCGAGAGTGGTACTGCAGTTGCGTTCATTGAT
ATGGCGAAGCGTATTAATCCATCTGCCCGTCTTATTTATAATGCTTCAGATAGTCTCAGTGCGATTA
ATGTAGCATGTTATATCGAGCGTGAATTTCAGCGGGTTGCTGCAAGTTTGGATGTGATCGCTGTTGT
TTCTCCTGCGATGACTAAAGAAATTCCTAGTCATGGCAATGTGTTTTATATCGGCCATGGGGTTCTT
CAAAATTTGAGTGAGCTTGGTGGTCCATCGCCATATGAGGGTGGATCCATGCCGTATCGGTTGGTT
CGATGTTGTTTGATCCGTTATTTTTTGTGGTTGCAGGGAAGGAGTTTCCACATATTACTTTTCATGT
TATCGGTTCAGGGATGGGGCGGCATCCTGATTATCCTGATAATGTCGTGGTTTACGGTGAGATGAAG
TACGTTGAAACTATTCGTTATATTAGGCATGCATCTTTCGGTATTGCGCCGTATGTGTCACAGCAGG
TTCCAGAATATTTAGCCGATAGTTCGATGAAACTGTTGCAGTACGACTTTTTCGGGTTACCGGCAGT
GTGTCCGCATGCGGTGGTCGGCTCTTATCCGACACGCTTTGGTTATACTCCAGGGAATGCTATTGAG
TTGGTGGCTGCGATCAAGCGTGCGTTGCAAGCGCCGCATCAGCAGTCTCGTCAATATTTGAGTTGGG
AAGAGGTTGTTGCTCGTGTTTTGGATCCTACTGCCTATGAGGGAACCCGGATATTACCTGCGGTGTG
A

FIGURE 1H

SEQ ID NO:18
GumKXf MVEYNGVHCYLWRTLVHPFNTRRSWLRGIEDMLFRWYVQHPPEILLQWIREADIILFESG 60
GumKXc --------------------------------MFRWYAAHPPKQLLDWMRESDVIVFESG 28
:****. ***: **:*:**:*:*:****

GumKXf TAVAFIDMAKRINPSARLIYNASDSLSAINVACYIEREFQRVAASLDVIAVVSPAMTKEI 120
GumKXc IAVAFIELAKRVNPAAKLVYRASDGLSTINVASYIEREFDRVAPTLDVIALVSPAMAAEV 88
*****::***:**:*:*:*.***.**:****.******:***.:*****:*****: *:

GumKXf PSHGNVFYIGHGVLQNLSELGGPSPYEGGIHAVSVGSMLFDPLFFVVAGKEFPHITFHVI 180
GumKXc ASRDNVFHVGHGVDHNLDQLGDPSPYAEGIHAVAVGSMLFDPEFFVVASKAFPQVTFHVI 148
.*:.***::**** :**.:**.**** *****:********* *****.* **::*****

GumKXf GSGMGRHPDYPDNVVVYGEMKYVETIRYIRHASFGIAPYVSQQVPEYLADSSMKLLQYDF 240
GumKXc GSGMGRHPGYGDNVIVYGEMKHAQTIGYIKHARFGIAPYASEQVPVYLADSSMKLLQYDF 208
********.* ***:******:.:** **:** ******.*:*** **************

GumKXf FGLPAVCPHAVVGSYPTRFGYTPGNAIELVAAIKRALQAPHQQSRQYLSWEEVVARVLDP 300
GumKXc FGLPAVCPNAVVGPYKSRFGYTPGNADSVIAAITQALEAPRVRYRQCLNWSDTTDRVLDP 268
********:****.* :********* .::***.:**:**: : ** *.*.:.. *****

GumKXf TAYEGTRILP-------------AV-- 312
GumKXc RAYPETRLYPHPPTAAPQLSSEAALSH 295
** **: * *:

SEQ ID NO.: 9
GTGTGGGCTTTTGGAGACTTTGTTGTGACGATCTCTGGCATTTCTTCCGATCCACCCTTCATTGAAA
CGATTGATACTCTGTTGCTTGGTGGGTTTCCGGTGTTATCAACCACGCCGTCTGCCTTTGTTGCTTC
GCTGTACCGTAGTTTGACTGAAGAAAGACCGCAGCGGGTATTTTTGCTAATACAAATTTTATCGTG
CAATGCCAGTCGCTCCGGGGGCGGATGTATTCGGAGCGGGTTTGTATCCTCAACGATGGGATAGGCA
TGGATTTGGCTGCGCTGTTCATTCATGGTCGTCGTTTTGTTAGTAATCTGAATGGTACTGATTTGAT
CCCGTATCTATGTCAGCACGCTCCACGTCCGTTGCGCTTTTTCTTGCTTGGTGCCAAGCCTGGTGTT
GCCGATATGGCGGCGCAGACGTTGTGTCAGTTGGGGCAATTAGTGGTCGGTACTTGTGATGGGTATG
CGCAGTTCATTGCGGCTGGTGAGGATTTAGTTGAGTCAATTAATGCCAGCGGTGCCGATGTGTTGTT
GGTTGCTTTGGGTAATCCTGTGCAGGAGCGTTGGATTCTTGATCATGATGTGCAGTTGCATACACCA
TTGGTTTTTGGTGTTGGTGCGTTGTTTGATTTTATGTCTGGTAATGTGCGGCGTGCGCCTTTATGGG
TACGTCGTGTACGTGGTGAATGGTTGTACCGATTGTTGTTGGAGCCAAGACGTTTATTTAAACGCTA
CAGTTGGGATCTGCTTCGGTTTTTTGGTGTGTGTTTATTACGGCGAAGGGGGGTGAATTAA

FIGURE 1I

SEQ ID NO:19 GumMXf MWAFGDFVVTISGISSDPPFIETIDTLLLGGFPVLSTTPSAFVASLYRSLTEERPQRVFF 60
GumMXc MHGQPAGVETATVSAATP---AQGVVIPLGGFPVLSTTQEAFALDLFHALAAHQPRRVFF 57
* .    * * :  :: *       .: ********** .**. .*:::*: .:*:****

GumMXf ANTNFIVQCQSLRGRMYSERVCILNDGIGMDLAALFIHGRRFVSNLNGTDLIPYLCQHAP 120
GumMXc ANTNFIVQCQALRARMQAPAVRIVNDGIGMDLAARLIHGRRFAGNLNGTDLIPYLCREAA 117
**********:**.** :  * *:********** :******..************:.*.

GumMXf RPLRFFLLGAKPGVADMAAQTLC-QLGQLVVGTCDGYAQFIAAGEDLVESINASGADVLL 179
GumMXc QPLKFFLLGGRPGVGKTAAATLGTLGQQVVGMCDGYGEFAAAGEGLAERINRSGADVLL 177
:**:*****.:***.. ** **    *** *** ****.:* ****.*.* ** *******

GumMXf VALGNPVQERWILDHDVQLHTPLVFGVGALFDFMSGNVRRAPLWVRRVRGEWLYRLLLEP 239
GumMXc VAFGNPLQERWILDHSEALQVPLVFGVGALLDFLSGTAKRAPNWVRRLHMEWMYRLLNEP 237
**:***:********.   *:.*********:**:**..:*** ****:: **:**** **

GumMXf RRLFKRYSWDLLRFFGVCLLRRRGVN 265
GumMXc RRLLKRYSWDLLVFFRTCLRAGKQLA 263
***:******** ** .**   : :



## Claims

1. An isolated nucleic acid molecule which comprises a nucleotide sequence which encodes at least one protein, the amino acid sequence of which is set forth at SEQ ID NOS: 11, 12, 13, 14, 15, 16, 17, 18, or 19, or a nucleotide sequence having at least 70% overall identity thereto.

2. The isolated nucleic acid molecule of claim 1, which encodes all of the proteins, the amino acid sequences of which are set forth at SEQ ID NOS: 11, 12, 13, 14, 15, 16, 17, 18 and 19.

3. The isolated nucleic acid molecule of claim 1, comprising at least one of the nucleotide sequences set forth at SEQ ID NOS: 1, 2, 3, 4, 5, 6, 7, 8 or 9.

4. The isolated nucleic acid molecule of claim 2, comprising the nucleotide sequence of SEQ ID NO: 10.

5. Expression vector comprising the isolated nucleic acid molecule of claim 1 or 2, operably linked to a promoter.

6. Bacterial cell other than *Xylella fastidiosa* or recombinant eukaryotic cell comprising the isolated nucleic acid molecule of claim 1 or 2.

7. Recombinant cell comprising the expression vector of claim 5.

8. Isolated protein comprising the amino acid sequence set forth in SEQ ID NOS: 11, 12, 13, 14, 15, 16, 17, 18 or 19.

9. Isolated protein consisting of the amino acid sequence set forth in SEQ ID NOS: 11, 12, 13, 14, 15, 16, 17, 18 or 19.

10. Antibody which specifically binds to the isolated protein of claim 9.

11. A process for making a compound of formula:
[glucose-glucose-mannose (Ac)-glucuronic acid]n
wherein n is a whole number from 1 to about 5000, comprising culturing a cell comprising the isolated nucleic acid molecule of claim 2, and isolating said compound following production thereof by said cell.

12. The process of claim 11, wherein said cell is *Xylella fastidiosa.*

13. The process of claim 11, wherein said cell is a recombinant cell.

14. A process for determining effect of a substance on a member of the XfGUM operon, comprising contacting a cell which comprises the isolated nucleic acid molecule of claim 2 with said substance, and determining effect of said substance on production of a compound of formula [glucose-glucose-mannose (Ac)-glucuronic acid]n
wherein n is a whole number from 1 to about 5000.

## Patentansprüche

1. Isoliertes Nukleinsäuremolekül, umfassend eine Nukleotidsequenz, die für mindestens ein Protein kodiert, dessen Aminosäuresequenz in den SEQ ID NOS: 11, 12, 13, 14, 15, 16, 17, 18 oder 19 dargelegt ist, oder eine Nukleotidsequenz, die dazu mindestens eine 70% Gesamt-Identität hat.

2. Isoliertes Nukleinsäuremolekül nach Anspruch 1, welches für alle Proteine kodiert, deren Aminosäuresequenzen in den SEQ ID NOS: 11, 12, 13, 14, 15, 16, 17, 18 und 19 dargelegt sind.

3. Isoliertes Nukleinsäuremolekül nach Anspruch 1, welches mindestens eine der in den SEQ ID NOS: 1, 2, 3, 4, 5, 6, 7, 8 oder 9 dargelegten Nukleotidsequenzen umfasst.

4. Isoliertes Nukleinsäuremolekül nach Anspruch 2, welches die Nukleotidsequenz gemäß SEQ ID NO: 10 umfasst.

5. Expressionsvektor, der das isolierte Nukleinsäuremolekül nach Anspruch 1 oder 2 umfasst, welches operativ mit einem Promoter verknüpft ist.

6. Bakterienzelle, mit Ausnahme von *Xylella fastidiosa,* oder eine rekombinante, eukaryotische Zelle, die das isolierte Nukleinsäuremolekül nach Anspruch 1 oder 2 umfasst.

7. Rekombinante Zelle, die den Expressionsvektor nach Anspruch 5 umfasst.

8. Isoliertes Protein, welches die in den SEQ ID NOS: 11, 12, 13, 14, 15, 16, 17, 18 oder 19 dargelegte Aminosäuresequenz umfasst.

9. Isoliertes Protein, welches aus der in SEQ ID NOS: 11, 12, 13, 14, 15, 16, 17, 18 oder 19 dargelegten Aminosäuresequenz besteht.

10. Antikörper, welcher spezifisch an das isolierte Protein nach Anspruch 9 bindet.

11. Verfahren zur Herstellung einer Verbindung nach Formel:
[Glucose-Glucose-Mannose (Ac)-Glucuronsäure]n,
wobei n eine ganze Zahl von 1 bis ungefähr 5000 ist, umfassend das Kultivieren einer Zelle, die das isolierte Nukleinsäuremolekül nach Anspruch 2 umfasst, und Isolieren der Verbindung im Anschluss an deren Produktion durch die Zelle.

12. Verfahren nach Anspruch 11, wobei die Zelle *Xylella fastidiosa* ist.

13. Verfahren nach Anspruch 11, wobei die Zelle eine rekombinante Zelle ist.

14. Verfahren zur Bestimmung der Wirkung einer Substanz auf ein Element des XfGUM Operons, umfassend das Kontaktieren einer Zelle, die das isolierte Nukleinsäuremolekül nach Anspruch 2 umfasst, mit der Substanz, und Bestimmen der Wirkung der Substanz auf die Produktion einer Verbindung nach Formel:
[Glucose-Glucose-Mannose (Ac)-Glucuronsäure]n,
wobei n eine ganze Zahl von 1 bis ungefähr 5000 ist.

## Revendications

1. Molécule d'acide nucléique isolée comprenant une séquence de nucléotides codant au moins une protéine dont la séquence d'acides aminés est indiquée dans les SEQ ID NO : 11, 12, 13, 14, 15, 16, 17, 18 ou 19, ou une séquence de nucléotides ayant une identité générale d'au moins 70% par rapport à celles-ci.

2. Molécule d'acide nucléique isolée de la revendication 1, qui code toutes les protéines dont la séquence d'acides aminés est indiquée dans les SEQ ID NO : 11, 12, 13, 14, 15, 16, 17, 18 et 19.

3. Molécule d'acide nucléique isolée de la revendication 1, comprenant au moins une des séquences de nucléotides indiquée dans les SEQ ID NO : 1, 2, 3, 4, 5, 6, 7, 8 ou 9.

4. Molécule d'acide nucléique isolée de la revendication 2, comprenant la séquence de nucléotides SEQ ID NO :10.

5. Vecteur d'expression comprenant la molécule d'acide nucléique isolée de la revendication 1 ou 2, lié de manière opérationnelle à un promoteur.

6. Cellule bactérienne autre que *Xylella fastidiosa* ou cellule eucaryote recombinée comprenant la molécule d'acide nucléique isolée de la revendication 1 ou 2.

7. Cellule recombinée comprenant le vecteur d'expression de la revendication 5.

8. Protéine isolée comprenant la séquence d'acides aminés indiquée dans les SEQ ID NO : 11, 12, 13, 14, 15, 16, 17, 18 ou 19.

9. Protéine isolée constituée de la séquence d'acides aminés indiquée dans les SEQ ID NO : 11, 12, 13, 14, 15, 16, 17, 18 ou 19.

10. Anticorps qui se lie de manière spécifique à la protéine isolée de la revendication 9.

11. Processus de réalisation d'un composé de formule :
[glucose-glucose-mannose (Ac)-acide glucuronique]n
dans lequel n est un nombre entier de 1 à environ 5000, comprenant la mise en culture d'une cellule comprenant la molécule d'acide nucléique isolée de la revendication 2 et l'isolation dudit composé après la production de celui-ci par ladite cellule.

12. Processus de la revendication 11, dans lequel ladite cellule est *Xylella fastidiosa.*

13. Processus de la revendication 11, dans lequel ladite cellule est une cellule recombinée.

14. Processus de détermination de l'effet d'une substance sur un élément de l'opéron XfGUM, comprenant la mise en contact d'une cellule qui comprend la molécule d'acide nucléique isolée de la revendication 2 avec ladite substance, et la détermination de l'effet de ladite substance sur la production d'un composé de formule
[glucose-glucose-mannose (Ac)-acide glucuronique]n
n étant un nombre entier de 1 à environ 5000.
